# EUROPÄISCHE PATENTSCHRIFT

(11) **EP 3 992 620 B1**
(45) Veröffentlichungstag und Bekanntmachung des Hinweises auf die Patenterteilung: **16.10.2024**
(21) Anmeldenummer: 21203209.8
(22) Anmeldetag: 18.10.2021
(51) Int. Cl.: G01N 23/046, G06T 11/00, A61B 6/58

(54) **COMPUTERIMPLEMENTIERTES VERFAHREN ZUR ERMITTLUNG MINDESTENS EINES FÜR EINE AUSWERTUNG VON MESSDATEN BENÖTIGTEN GEOMETRISCHEN PARAMETERS**
COMPUTER-IMPLEMENTED METHOD FOR DETERMINING AT LEAST ONE GEOMETRIC PARAMETER REQUIRED FOR EVALUATION OF MEASUREMENT DATA
PROCÉDÉ MIS EN OEUVRE PAR ORDINATEUR DESTINÉ À LA DÉTERMINATION D'AU MOINS UN PARAMÈTRE GÉOMÉTRIQUE NÉCESSAIRE POUR UNE ÉVALUATION DES DONNÉES DE MESURE

(30) Priorität: 28.10.2020 DE 102020128288
(43) Veröffentlichungstag der Anmeldung: 04.05.2022
(73) Patentinhaber: Volume Graphics GmbH, 69115 Heidelberg (DE)
(72) Erfinder: POLIWODA, Christoph, 69115 Heidelberg (DE); SCHÜLLER, Sören, 69115 Heidelberg (DE); FLEßNER, Matthias, 69115 Heidelberg (DE); GÜNTHER, Thomas, 69115 Heidelberg (DE)
(74) Vertreter: Patentanwälte Olbricht Buchhold Keulertz

(56) Entgegenhaltungen:
- DE-A1- 102009 049 818
- DE-A1- 102015 225 130
- DE-A1- 102016 214 062
- DE-A1- 102017 205 113
- DE-B3- 102017 201 164
- US-A- 5 550 376
- US-A1- 2007 122 020

## Beschreibung

Die Erfindung betrifft ein computerimplementiertes Verfahren zur Ermittlung mindestens eines für eine Auswertung von Messdaten benötigten geometrischen Parameters.

Das innere von Objekten oder Lebewesen kann mittels Durchstrahlungsbildern, wie z. B. den Bildern einer computertomographischen Messung, dargestellt werden. Um z. B. eine computertomographische Messung durchführen zu können, muss die geometrische Kalibrierung bekannt sein, die sich aus der geometrischen Positionierung und Orientierung der Strahlungsquelle, des Detektors und des zu messenden Objekts zueinander für jedes einzelne aufgenommene Durchstrahlungsbild ergibt. In einem einzelnen Durchstrahlungsbild kann die geometrische Positionierung und Orientierung der Strahlungsquelle, des Detektors und des Bauteils zueinander, insbesondere die Pose der Röntgenquelle und des Detektors bzgl. dem Bauteil bzw. Drehtisch, als Aufnahmegeometrie bezeichnet werden. Die geometrische Kalibrierung wird von der Rekonstruktion benötigt, bei der aus einer Vielzahl von Durchstrahlungsbildern mit unterschiedlichen Aufnahmegeometrien eines Objekts Volumendaten des Objekts ermittelt werden. Fehler der geometrischen Kalibrierung verursachen Bildfehler in den rekonstruierten Volumendaten, z. B. unscharfe Kanten, Doppelkanten oder Verzerrungen, usw., und somit Messfehler bei der Durchführung von dimensionellen Messungen oder anderen Analysen wie einer Defektanalyse.

Für eine geometrische Kalibrierung ist die Kenntnis der geometrischen Parameter der Vorrichtung zur Durchführung der durchstrahlenden Messung am Objekt nötig. Im allgemeinen Fall sind neun Parameter bzw. Freiheitsgrade oder Degrees of Freedom, auch DoF genannt, für jedes einzelne Durchstrahlungsbild nötig. Dies sind drei translatorische und drei rotatorische Parameter bzgl. des Detektors und drei translatorische Parameter bzgl. der Strahlungsquelle, wenn das starre zu messende Objekt als Bezug gewählt wird. Andere Definitionen sind allerdings ebenfalls möglich. Auf diese Weise kann eine Zuordnung von Pixeln von Durchstrahlungsbildern, die zweidimensionalen Projektionen entsprechen, zu Koordinaten im Raum bzw. Voxeln von dreidimensionalen Volumendaten hergestellt werden. Dieser allgemeine Fall, z. B. bei komplexen Trajektorien von Roboter-Computertomographen, benötigt eine Bestimmung der Parameter für jedes Durchstrahlungsbild. Dabei werden für jedes Durchstrahlungsbild lokale Parameter ermittelt.

Bei bestimmten Trajektorien, z. B. der Kreistrajektorie einer axialen Computertomographie oder bei der Spiral-Computertomographie, kann es weniger Parameter geben. In diesem Fall werden globale Parameter bestimmt, aus denen sich anhand der Vorgaben der Trajektorie die lokalen Parameter für jedes einzelne Durchstrahlungsbild errechnen lassen. Dies schließt nicht aus, dass für die globalen Parameter für jedes einzelne Durchstrahlungsbild Abweichungen von einer idealen Aufnahmegeometrie registriert werden, die in den lokalen Parametern bereits direkt berücksichtigt sind.

Allerdings können insbesondere Roboter-Computertomographen und C-Arm-Computertomographen allgemein den Detektor und die Strahlungsquelle nicht genau positionieren. Dies betrifft nicht nur systematische Abweichungen, sondern auch zufällige, so dass die Abweichungen von einer idealen Aufnahmegeometrie somit bei jeder Messung unterschiedlich sein können. Auch normale axiale Computertomographen weisen neben den systematischen auch zufällige Abweichungen auf, z. B. durch thermische Effekte, ein Taumeln der Drehachse, langsame Drifts des Spots in der Strahlungsquelle oder der Achsen, ein Pitting der Röhre, Vibrationen, Abweichungen bzw. Fehler der Achsen. Weiter können Bewegungen des zu messenden Objekts oder des Lebewesens während der durchstrahlenden Messung eine Ursache sein.

Aus der US 7,186,023 B ist bekannt, ein dediziertes Normal, vorab oder im Nachhinein in allen benötigten Aufnahmegeometrien zu durchstrahlen. Dies kann als sog. Offline-Kalibrierung bezeichnet werden. Anhand der Position des Normals in den einzelnen Durchstrahlungsbildern kann jeweils die Aufnahmegeometrie zurückgerechnet werden. Allerding lässt über die Zeit die Genauigkeit der Kalibrierung nach, da die Vorrichtungen zur Durchführung der durchstrahlenden Messung, insbesondere Roboter-Computertomographen, nicht perfekt stabil sind. Diese Kalibrierung bzw. dieser Einmessvorgang müsste daher regelmäßig wiederholt werden. Dies ist zeitaufwendig. Zudem kann es vorkommen, dass Drifteffekte auch während einer Messung, direkt nach dem Kalibrieren, auftreten. Bei flexiblen bzw. adaptiven Trajektorien ist es zudem häufig nicht möglich, vorab eine Einmessung durchzuführen, da die zu verwendende Trajektorie noch nicht bekannt ist.

Weiter ist bekannt, aus den Messdaten selbst eine Kalibrierung abzuleiten. Dies kann als Online-Kalibrierung bezeichnet werden. Bei einer Online-Kalibrierung fällt die Unsicherheit der Übertragung einer Offline-Kalibrierung auf den Zeitpunkt der Messung weg. Allerdings sind diese Kalibrierungen an sich in der Regel ungenauer ist, da kein dediziertes Normal mit bekannter Geometrie verwendet werden kann. Weiter sind diese Vorgehensweisen rechenaufwändig und komplex.

DE 10 2009 049818 A1 offenbart ein Verfahren zur Online-Kalibrierung und Ermittlung von für die Auswertung der Messung benötigten geometrischen Parametern, wobei aus Messdaten einer durchstrahlenden Messung Referenzbereiche (Kanten, Ecken, Bohrungen, etc.) identifiziert werden, und die geometrischen Parameter aus einer bekannten digitalen Bauteildarstellung und aus den Messdaten der durchstrahlenden Messung ermittelt werden.

Aufgabe der Erfindung ist es daher ein computerimplementierte Verfahren zu schaffen, das weniger Rechenleistung als der Stand der Technik benötigt und ohne großen Aufwand anwendbar ist.

Hauptmerkmale der Erfindung sind in Anspruch 1 angegeben. Ausgestaltungen sind Gegenstand der Ansprüche 2 bis 13. In einem ersten Aspekt betrifft die Erfindung ein computerimplementiertes Verfahren zur Ermittlung mindestens eines für eine Auswertung von Messdaten benötigten geometrischen nach Anspruch 1. Mit der Erfindung wird eine Online-Kalibrierung durchgeführt, bei der hochgenaue geometrische Parameter ermittelt werden. Dazu wird in der digitalen Bauteildarstellung, die aus einer Rekonstruktion der Messdaten bzw. der Durchstrahlungsbilder erzeugt wird, mindestens ein Referenzbereich identifiziert. Ein Referenzbereich umfasst lediglich einen Teil des Bauteils, nicht das gesamte Bauteil. Lediglich auf Basis dieses mindestens einen Referenzbereichs wird ein geometrischer Parameter ermittelt, der für die Auswertung der ermittelten Messdaten, d. h. z. B. für eine dimensionelle Messung, benötigt wird. Hierfür werden in der Bauteilgeometrie, d. h. in den Durchstrahlungsbildern oder den Volumendaten der Rekonstruktion bzw. deren Segmentierung, Bereiche bzw. Geometrien im oder am Bauteil ermittelt, die möglichst gut geeignet sind, um mit ihnen eine geometrische Kalibrierung durchzuführen. Im Idealfall wird die Kalibrierung dann berechnet wie bei einer Messung eines dedizierten Normals, ohne dass der Scan eines solchen benötigt wird. Dies reduziert den Aufwand der Kalibrierung. Weiter wird weniger Rechenleistung benötigt als bei bekannten Online-Kalibrierverfahren. Es werden gezielt geeignete Referenzbereiche gesucht, anhand der die Kalibrierung umgesetzt wird bzw. die geometrischen Parameter ermittelt werden. Die gezielte Verwendung dieser Referenzbereiche reduziert die benötigte Rechenzeit, insbesondere pro Iteration, bzw. ermöglicht ggf. höhere Genauigkeiten im Vergleich zu bekannten Online-Kalibrierverfahren.

Geht man von einer bekannten geometrischen Kalibrierung aus, kann das Verfahren zum Beispiel weiter dazu verwendet werden, Parameter anderer geometrischer Effekte zu ermitteln und diese Effekte auf diese Weise zu korrigieren. Bei diesen Effekten kann es sich um Abweichungen des Detektors von der Nominalgestalt und ein nicht-rigides Verhalten, d. h. Verformungen, des zu messenden Objekts bzw. Bauteils während der Messung handeln.

In einem Beispiel können Parameter von zwei von drei geometrischen Effekten bekannt sein und die Parameter des dritten geometrischen Effekts ermittelt werden. Denkbar ist ebenfalls, die Parameter von zwei oder sogar aller drei geometrischer Effekte gleichzeitig zu ermitteln.

Als Referenzbereiche werden ausgewählte Geometrien des Messobjekts bzw. ausgewählte Teilbereiche der digitalen Bauteildarstellung, insbesondere der Volumendaten, bezeichnet, die diese Geometrien aufweisen. In einem Beispiel können die Referenzbereiche auch mit Hilfe von vorhandenen Vorwissen über die Bauteilgeometrie identifiziert werden. Es können auch bereits vor Durchführung der Messung Referenzbereiche ermittelt werden.

Weiter können beispielsweise die Referenzbereiche in den Durchstrahlungsbildern bzw. den Messdaten ermittelt werden, z. B. Bereiche mit hohem Kontrast und/oder scharfen Kanten. Es werden die Bereiche der Durchstrahlungsbilder verwendet, in denen die Referenzbereiche, d. h. die Geometrien des Bauteils bzw. die ausgewählten Teilbereiche der Volumendaten der digitalen Bauteildarstellung, abgebildet werden. Dabei kann es zum Beispiel denkbar sein, dies für jede Projektion einzeln durchzuführen.

Die Auswertung der ermittelten Messdaten kann insbesondere eine Rekonstruktion beinhalten.

Die für die Auswertung benötigten geometrischen Parameter können beispielsweise Parameter sein, die die Aufnahmegeometrie, die Abweichungen des Detektors von der Nominalgestalt oder die nicht-rigides Verhalten des Bauteils während der Messung beschreiben.

Gemäß einem Beispiel kann der mindestens eine geometrische Parameter eine für die durchstrahlende Messung des Bauteils verwendete Aufnahmegeometrie beschreiben.

Dies kann als geometrische Kalibrierung einer Vorrichtung zur Durchführung der durchstrahlenden Messung, insbesondere eines Computertomographie-Systems, bezeichnet werden. Eine Möglichkeit der Parametrisierung ist die Beschreibung jedes Durchstrahlungsbilds der Messdaten durch neun Parameter bzw. Freiheitsgrade.

Weiter kann das Verfahren beispielsweise zwischen dem Schritt: Ermitteln der Messdaten, und dem Schritt: Identifizieren von Bereichen in der digitalen Bauteildarstellung und/oder in der Bauteilgeometrie als Referenzbereiche, folgenden Schritt aufweisen: Ermitteln einer vorläufigen Rekonstruktion des Bauteils als digitale Bauteildarstellung aus zumindest einem Teil der ermittelten Messdaten mittels mindestens eines vordefinierten geometrischen Start-Parameters, wobei im Schritt: Identifizieren von Bereichen in der digitalen Bauteildarstellung und/oder in der Bauteilgeometrie als Referenzbereiche, die Bereiche in der digitalen Bauteildarstellung und/oder in einer auf der digitalen Bauteildarstellung basierenden Segmentierung identifiziert werden.

Die Referenzbereiche können mit Hilfe einer vorläufigen Rekonstruktion bzw. digitalen Bauteildarstellung oder einer auf dieser vorläufigen Rekonstruktion durchgeführten Segmentierung identifiziert, wobei die vorläufige Rekonstruktion auf Basis mindestens eines Teils der Durchstrahlungsbilder bzw. der Messdaten durchgeführt wird. Dies kann beispielsweise bedeuten, dass die Ermittlung der Messdaten noch nicht abgeschlossen ist, jedoch die bereits vorhandenen Messdaten oder ein Teil der vorhandenen Messdaten für eine vorläufige Rekonstruktion verwendet werden.

Dabei kann das Verfahren nach dem Schritt: Ermitteln mindestens eines geometrischen Parameters, beispielsweise weiter folgende Schritte aufweisen: Wiederholen der folgenden Schritte solange ein vordefiniertes Abbruchkriterium nicht erfüllt wird: Ermitteln einer weiteren vorläufigen Rekonstruktion des Bauteils als digitale Bauteildarstellung aus zumindest einem Teil der ermittelten Messdaten mittels des mindestens einen geometrischen Parameters; Identifizieren von Bereichen in der digitalen Bauteildarstellung als Referenzbereiche; Ermitteln mindestens eines weiteren geometrischen Parameters, der den mindestens einen geometrischen Parameter ersetzt und für eine Auswertung der Messdaten benötigt wird, mittels der Referenzbereiche.

Da die geometrischen Parameter nun genauer als vor den Wiederholungen sind, weist die neue vorläufige digitale Bauteildarstellung bzw. Rekonstruktion eine höhere Qualität auf. Damit kann die mindestens eine Referenzgeometrie besser ausgewählt werden. Weiter ist besseres Wissen über die Referenzbereiche bekannt, z. B. die relative Positionierung von Referenzbereichen bzw. der in ihnen enthaltenen Geometrien zueinander, welches in der neuen Iteration genutzt wird. Der Wiederholungsschritt kann abgebrochen werden, wenn z. B. eine definierte Anzahl von Iterationen erreicht ist, die geometrischen Parameter sich nicht mehr signifikant zwischen Iterationsschritten ändern, d. h. sie konvergieren, oder die Messdaten oder die aus ihnen rekonstruierten Volumendaten ein definiertes Qualitätsmaß erfüllen.

Gemäß der Erfindung weist der Schritt: Ermitteln mindestens eines geometrischen Parameters, mindestens einen der folgenden Unterschritte auf: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters; und/oder Simulieren einer durchstrahlenden Messung an der digitalen Bauteildarstellung und/oder einer auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters; und/oder Variieren mindestens eines geometrischen Parameters der digitalen Bauteildarstellung in mindestens einer Projektion der digitalen Bauteildarstellung, Ermitteln mindestens eines Qualitätsparameters einer die mindestens eine Projektion verwendenden Rekonstruktion des Bauteils, Wiederholen des Variierens des mindestens einen geometrischen Parameters und der Ermittlung mindestens eines Qualitätsparameters bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter.

Bei der Ermittlung der Referenzbereiche wird erfindungsgemäß ermittelt, für welchen der drei Unterschritte zur Bestimmung der geometrischen Parameter diese jeweils eingesetzt werden können. Kleine, kugelförmige Einschlüsse sind beispielsweise für alle drei oben erläuterten Unterschritte prinzipiell vorteilhaft. Unregelmäßig geformte Einschlüsse sind für die letzten beiden oben erläuterten Unterschritte vorteilhaft. Bereiche eines Materialübergangs mit hohem Kontrast sind für den letzten Unterschritt: Variieren mindestens eines geometrischen Parameters der digitalen Bauteildarstellung in mindestens einer Projektion der digitalen Bauteildarstellung, Ermitteln mindestens eines Qualitätsparameters einer die mindestens eine Projektion verwendenden Rekonstruktion des Bauteils, Wiederholen des Variierens des mindestens einen geometrischen Parameters und der Ermittlung mindestens eines Qualitätsparameters bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter, vorteilhaft.

In einem Beispiel des Unterschritts: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, können abgesehen von einer globalen Translation und Rotation, die zusammen sechs Freiheitsgrade ausmachen, nun die Positionen der Referenzbereiche zueinander ermittelt werden und somit auch die Abstände zueinander. Die Positionen der Referenzbereiche können somit in einem eigenen Koordinatensystem beschrieben werden. Mit Hilfe dieser Informationen können die geometrischen Parameter ermittelt werden, für die die projizierten Positionen der Referenzbereiche und deren Positionen in der vorläufigen digitalen Bauteildarstellung, vorzugsweise möglichst, konsistent sind. Im Falle der geometrischen Kalibrierung werden damit anhand der projizierten Positionen die jeweilige Pose, d. h. die Position und Ausrichtung von Detektor und Position von Röntgenquelle bezüglich der Referenzbereiche und somit des definierten Koordinatensystems, ermittelt. Diese geometrische Information kann dann für eine korrekte Rekonstruktion einer digitalen Bauteildarstellung verwendet werden, die die vorläufige digitale Bauteildarstellung ersetzen kann.

Für dieses Beispiel ist es vorteilhaft, als Referenzbereiche Geometrien des Bauteils zu verwenden, deren projizierte Positionen in den Durchstrahlungsbildern gut erkennbar bzw. lokalisierbar sind. Ein Beispiel hierfür sind kugelförmige Geometrien. Aufgrund der Freiheitsgrade, ggf. jedes einzelnen Durchstrahlungsbilds, ist dabei eine gewisse Mindestanzahl von Geometrien nötig. Weiter ist vorteilhaft, drei, vier oder sogar mehr Referenzbereiche zu verwenden, um eine genaue geometrische Kalibrierung zu erreichen. Weiter kann zum Beispiel eine vergleichsweise große Anzahl von Referenzbereichen, z. B. mindestens 8, 20, 50 oder 100, verwendet werden. Dann ist es unproblematisch, wenn die projizierten Positionen der einzelnen Referenzbereiche in den Durchstrahlungsbildern vergleichsweise ungenau lokalisiert werden können, sich teilweise gegenseitig überdecken oder sie nicht in allen Durchstrahlungsbildern sichtbar sind, da dies durch die verbesserte Statistik wieder ausgeglichen werden kann.

Weiter können in einem weiteren Beispiel Referenzbereiche in einzelnen Projektionen nicht berücksichtigt werden, wenn sich diese in den Durchstrahlungsbildern gegenseitig überlappen bzw. überdecken oder sich zumindest sehr nahe sind oder sich in einem Bereich der Projektion befinden, in denen die Messwerte, im Falle der Computertomographie Grauwerte, bzw. der Hintergrund der Messwerte, z. B. ein Grauwerthintergrund, vergleichsweise inhomogen sind. Dies erschwert eine Ermittlung der genauen Position der Referenzgeometrie in der Projektion.

Im Allgemeinen sind nichtlineare Gleichungen zu lösen, um die geometrischen Parameter zu ermitteln. Eine iterative Lösung mit Hilfe des Levenberg-Marquardt-Algorithmus ist zum Beispiel denkbar. Allerdings kann die ermittelte Lösung in einem lokalen Minimum landen.

Für eine axiale Aufnahmegeometrie kann zum Beispiel ein Ellipsen-Fit der Bahn einer Referenzgeometrie in den Durchstrahlungsbildern stabiler sein, sofern keine freien Trajektorien verwendet werden.

Um eine komplette geometrische Kalibrierung durchzuführen, kann der Unterschritt: Ermitteln mindestens eines geometrischen Parameters, mindestens einen der folgenden Unterschritte aufweisen: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, für alle Durchstrahlungsbilder durchgeführt werden.

Im Unterschritt: Simulieren einer durchstrahlenden Messung an der digitalen Bauteildarstellung und/oder einer auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters, kann zum Beispiel der Satz von geometrischen Parametern für das betrachtete Durchstrahlungsbild gesucht werden, für den das simulierte Durchstrahlungsbild der simulierten Messdaten und das real gemessene Durchstrahlungsbilder der ermittelten Messdaten für die Referenzbereiche die beste Übereinstimmung aufweisen. Hierfür können Ähnlichkeitsmaße verwendet werden, z. B. eine Korrelation von Pixel-Grauwerten im Fall der Computertomographie. Denkbar ist beispielsweise, jeweils eine Gradienten-Darstellung zu vergleichen. Das simulierte Durchstrahlungsbild kann z. B. mit Hilfe von Ray-Casting-basierten Methoden oder von Vorwärtsprojektionen durchgeführt werden. Um eine komplette geometrische Kalibrierung durchzuführen, kann dieser Unterschritt für alle Durchstrahlungsbilder durchgeführt werden.

Es werden weiterhin entsprechende Referenzbereiche ermittelt, die eine projektionsbasierte Korrektur ermöglichen. Anstatt wie bei dem Unterschritt: Ermitteln mindestens eines geometrischen Parameters, mindestens einen der folgenden Unterschritte aufweisen: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, allerdings "analytisch" die Position der Referenzgeometrie in 3D mit der projizierten Position in 2D abzugleichen, arbeitet der Unterschritt Simulieren einer durchstrahlenden Messung an der digitalen Bauteildarstellung und/oder einer auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters, auf den Bilddaten. Eine digitale Durchstrahlung wird mit dem real gemessenen Durchstrahlungsbild verglichen und es werden die geometrischen Parameter gesucht, die eine größte Übereinstimmung bewirken. Dies wird nur auf den Bereichen der ermittelten, z. B. markanten, Referenzbereiche durchgeführt. Dies verringert die Rechenzeit deutlich.

Gemäß einem Beispiel des Unterschritts: Variieren mindestens eines geometrischen Parameters der digitalen Bauteildarstellung in mindestens einer Projektion der digitalen Bauteildarstellung, Ermitteln mindestens eines Qualitätsparameters in mindestens einem Referenzbereich einer die mindestens eine Projektion verwendenden Rekonstruktion des Bauteils, Wiederholen des Variierens des mindestens einen geometrischen Parameters und der Ermittlung mindestens eines Qualitätsparameters in mindestens einen Referenzbereich bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter, wird der Satz geometrischer Parameter für das mindestens eine betrachtete Durchstrahlungsbild gesucht, bei denen die Volumendaten der Referenzbereiche die besten Eigenschaften aufweisen. Diese Eigenschaften können z. B. Kontrast, die Entropie, die Auflösung bzw. die Schärfe von Kanten/Materialübergängen sein, da fehlerhafte geometrische Parameter sich üblicherweise negativ auf diese Eigenschaften auswirken. Dies kann jeweils anhand der Volumendaten selbst und/oder anhand eines Grauwerthistogramms beurteilt werden. Eine Optimierung kann zudem dahingehend durchgeführt werden, dass sich im Grauwerthistogramm möglichst gut getrennte Peaks der einzelnen Materialien auszeichnen.

In einem Beispiel mit für eine geometrische Kalibrierung wirkt sich eine einzelne Projektion bzw. Durchstrahlungsbild nur schwach auf die Volumendaten aus. Daher werden vorteilhafterweise die geometrischen Parameter aller Projektionen gemeinsam optimiert, d. h. die globalen Parameter, da es ansonsten sehr viele Freiheitsgrade gäbe.

Für die weitere Optimierung der Qualität der Volumendaten werden die Bereiche des Volumens, das hierfür ausgewertet wird, intelligent ausgewählt. Diese Bereiche sind die Referenzbereiche. Auf diese Weise kann die Optimierung schneller und zielgerichteter und somit ggf. auch genauer durchgeführt werden.

Alle drei oben beschriebenen Unterschritte sind prinzipiell geeignet, die geometrischen Parameter der drei oben genannten geometrischen Effekte zu ermitteln. Auch hier ist die geometrische Kalibrierung der in der Praxis wichtigste Effekt. Dabei können beispielsweise mehrere der oben beschriebenen Unterschritte kombiniert bzw. gleichzeitig verwendet werden. Einige der Referenzbereiche können zum Beispiel nur für einen der Unterschritte verwendet werden, andere für zwei oder drei der oben beschriebenen Unterschritte, je nach Eignung.

Der Unterschritt: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, ist tendenziell besser geeignet, wenn als Referenzbereiche einfache und in den Projektionen klar erkennbare Strukturen, wie z. B. Kugeln oder kleine Einschlüsse, verwendet werden, die exakt lokalisiert werden können. Der Unterschritt: Simulieren einer durchstrahlenden Messung an der digitalen Bauteildarstellung und/oder einer auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters, ist tendenziell besser geeignet, wenn komplexere Strukturen als Referenzbereiche verwendet werden.

In einem Beispiel der geometrischen Kalibrierung sind die beiden zuerst genannten Unterschritte am besten für Roboter-Computertomographen bzw. freie Trajektorien tendenziell am besten geeignet. Ebenfalls um die Abweichungen der einzelnen Projektionen von der zugehörigen idealen Trajektorie zu ermitteln. Um die globalen Parameter einer konventionellen axialen Computertomographie oder Helix-Computertomographie zu ermitteln sind alle drei Unterschritte prinzipiell geeignet.

Dabei können zum Beispiel im Unterschritt: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters; Geometrien der Referenzbereiche beim Ermitteln der projizierten Positionen berücksichtigt werden.

Die Berücksichtigung der Geometrien der Referenzbereiche betrifft insbesondere eine Abweichung von einer kugelförmigen Geometrie. So kann z. B. das Wissen über die dreidimensionale Geometrie des Referenzbereichs, z. B. aus einer Defektanalyse, verwendet werden, um entsprechende Parameter, z. B. einen Schwerpunkt eines Extremums der Werte der Pixel eines Durchstrahlungsbilds oder eine Umrandung der Abbildung des Referenzbereichs, in der zweidimensionalen Projektion vorherzusagen. Diese können daraufhin besser oder exakter zu identifizieren sein. Auf diese Weise wird die Genauigkeit der Zuordnung der Positionen der Geometrien in den Durchstrahlungsbildern bzw. der Projektionen untereinander bzw. zu der Position im in der digitalen Bauteildarstellung erhöht. Dies kann zum Beispiel umgesetzt werden, in dem die Hauptachsen einer Geometrie ermittelt werden und damit die Auswirkungen auf die Projektion ermittelt bzw. modelliert werden.

Weiter können zum Beispiel im Unterschritt: Simulieren einer durchstrahlenden Messung an der digitalen Bauteildarstellung und/oder der auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters; die simulierten Messdaten lediglich für Bereiche in der digitalen Bauteildarstellung erzeugt werden, die Referenzbereiche aufweisen.

Mit anderen Worten werden die simulierten Durchstrahlungen nur in den Bereichen der Referenzbereiche und ggf. einer kleinen Umgebung um die Referenzbereiche herum berechnet und mit den ermittelten Durchstrahlungsbildern aus den Messdaten abgeglichen. Damit wird die benötigte Rechenleistung verringert. Die Ableitung bzw. Gradientendarstellung oder andere Darstellungen, z. B. nach Anwendung eines Sobel-Operators, der Projektionen können dabei zum Beispiel betrachtet bzw. verglichen werden. Weiter kann die durchstrahlende Messung lediglich für einen kegelförmigen Bereich des Bauteils simuliert werden, wobei der kegelförmige Bereich den Referenzbereich beinhaltet. Es werden dann lediglich für die Bereiche des Bauteils simulierte Messdaten erzeugt, in denen geometrische Parameter für die Auswertung benötigt werden. Dies verringert die benötigte Rechenleistung weiter.

In einem anderen Beispiel können im Unterschritt: Variieren mindestens eines geometrischen Parameters der digitalen Bauteildarstellung in mindestens einer Projektion der digitalen Bauteildarstellung, Ermitteln mindestens eines Qualitätsparameters in mindestens einem Referenzbereich einer die mindestens eine Projektion verwendenden Rekonstruktion des Bauteils, Wiederholen des Variierens des mindestens einen geometrischen Parameters und der Ermittlung mindestens eines Qualitätsparameters in dem mindestens einen Referenzbereich bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter; lediglich die Bereiche der digitalen Bauteildarstellung mittels der variierten geometrischen Parameter in der digitalen Bauteildarstellung neu rekonstruiert und analysiert werden, die Referenzbereiche aufweisen.

Durch das wiederholte Variieren der geometrischen Parameter und dem Rekonstruieren und Analysieren bei der Ermittlung des optimierten geometrischen Parameters können die korrekten geometrischen Parameter identifiziert werden. Da nur kleine Bereiche rekonstruiert werden müssen, geht dies schneller bzw. es sind mehr Iterationen in gleicher Zeit möglich.

Der Schritt: Identifizieren von Bereichen in der digitalen Bauteildarstellung und/oder in der Bauteilgeometrie als Referenzbereiche, kann beispielsweise den folgenden Unterschritt aufweisen: Segmentieren von Grenzflächen der digitalen Bauteildarstellung.

In diesem Beispiel kann die Segmentierung sowohl äußere Grenzflächen als auch innere Grenzflächen umfassen. Innere Grenzflächen können innere Oberflächen des Bauteils oder im Bauteil sein, z. B. von Einschlüssen, Poren oder Defekten sein, die sehr klein sein können. Vorteilhafterweise kann die Segmentierung in einem Beispiel subvoxelgenau durchgeführt werden. Die Segmentierung kann weiter zum Beispiel auf Basis der Volumendaten der digitalen Bauteildarstellung, jedoch ebenfalls auf Basis der Messdaten bzw. der Durchstrahlungsbilder durchgeführt werden. Weiter ist beispielsweise denkbar, für die Identifizierung der Referenzbereiche sowohl die Segmentierung als auch die Volumendaten gemeinsam zu betrachten. Dabei können zum Beispiel kleine kugelförmige Geometrien mit hoher Absorption und/oder gutem Kontrast in den Volumendaten der digitalen Bauteildarstellung verwendet werden.

Weiter können zum Beispiel im Schritt: Identifizieren von Bereichen in der digitalen Bauteildarstellung und/oder in der Bauteilgeometrie als Referenzbereiche, Bereiche identifiziert werden, in denen Material- und/oder Luft-Einschlüsse angeordnet sind.

Die Material- und/oder Luft-Einschlüsse sind im Regelfall vorab nicht bekannt und werden erst mit Hilfe einer geeigneten Analyse der Messdaten oder der Volumendaten der digitalen Bauteildarstellung identifiziert. Dies kann beispielsweise eine Analyse sein, welche eine Defektmaske ermittelt. Vorteilhafterweise können zum Beispiel nur Material-Einschlüsse verwendet werden, da diese einen besseren Kontrast als Luft-Einschlüsse aufweisen. Dies gilt insbesondere für Metall-Einschlüsse. Insbesondere ist dieses Merkmal in Kombination mit dem Unterschritt: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, vorteilhaft.

Gemäß einem weiteren Beispiel können im Schritt: Identifizieren von Bereichen in der digitalen Bauteildarstellung und/oder in der Bauteilgeometrie als Referenzbereiche, Bereiche identifiziert werden, die eine Geometrie aufweisen, die kugelförmig ist oder unter Berücksichtigung eines vordefinierten Toleranzbereiches als kugelförmig klassifiziert wird.

Insbesondere bzgl. Material- und/oder Luft-Einschlüssen kann dies vorteilhaft sein, da die Einschlüsse kugelförmige Geometrien aufweisen, die in Projektionen bzw. Durchstrahlungsbildern leicht lokalisiert werden können. Weiter werden sie in verschiedenen Durchstrahlungsrichtungen sehr ähnlich in den Projektionen abgebildet. Es ist eine Analyse der Geometrie der Material- und/oder Luft-Einschlüsse nötig, um diejenigen mit der entsprechenden Geometrie zu identifizieren. Dies kann beispielsweise auf Basis einer Defektmaske geschehen, die zum Beispiel aus einer Defektanalyse erhalten werden kann. Insbesondere in der additiven Fertigung kann es viele Materialeinschlüsse geben. Um die Position von kugelförmigen Geometrien in den Projektionen, möglichst subpixelgenau, zu ermitteln, kann zum Beispiel der Massenschwerpunkt der Abbildung der Geometrie in der Projektion ermittelt werden. Insbesondere ist dieses Merkmal in Kombination mit dem Unterschritt: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, vorteilhaft.

Im Schritt: Identifizieren von Bereichen in der digitalen Bauteildarstellung und/oder in der Bauteilgeometrie als Referenzbereiche, können weiter zum Beispiel Bereiche identifiziert werden, die ein Objekt einer Baugruppe im Bauteil sind und einen Absorptionskoeffizienten aufweisen, der innerhalb eines vordefinierten Intervalls angeordnet ist.

Dies können beispielsweise Schrauben, Nägel, Bolzen, Nieten, Metallkugeln, Kabel oder Teile eines Steckers sein. Es werden vorzugsweise kleine Objekte von Baugruppen gewählt. Es ist vorteilhaft, wenn die Absorptionskoeffizienten dieser Objekte vergleichsweise hoch sind. Insbesondere ist dieses Merkmal in Kombination mit dem Unterschritt: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, vorteilhaft.

Weiter können im Schritt: Identifizieren von Bereichen in der digitalen Bauteildarstellung und/oder in der Bauteilgeometrie als Referenzbereiche, zum Beispiel Bereiche identifiziert werden, die in einem Volumen der Bauteilgeometrie angeordnet sind, in dem keine Referenzbereiche oder eine Anzahl an Referenzbereichen, die kleiner als ein vordefinierter Grenzwert ist, identifiziert wurden.

Die erreichbare Genauigkeit der geometrischen Kalibrierung kann auf diese Weise erhöht werden, da sich z. B. einige Fehler der geometrischen Kalibrierung unterschiedlich in verschiedenen Regionen des das Bauteil aufweisenden Messvolumens äußern. Insbesondere in Kombination mit dem Unterschritt: Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters, werden stabilere Ergebnisse erhalten, wenn die Geometrien möglichst verteilt bzw. weit voneinander entfernt angeordnet sind.

Beispielgemäß können im Schritt: Ermitteln mindestens eines geometrischen Parameters, der für eine Auswertung der Messdaten benötigt wird, mittels der Referenzbereiche, Sicherheitsparameter der Referenzbereiche berücksichtigt werden.

Der Sicherheitsparameter kann angeben, wie sicher eine Position des ihm zugeordneten Referenzbereichs ist. Referenzbereiche, die aufgrund ihres Sicherheitsparameters als unsicher beurteilt wurden, können zum Beispiel geringer gewichtet werden, z. B. bei einer gewichteten Anpassung. Auf diese Weise wird die Gefahr einer fehlerhaften Anpassung minimiert.

In allen oben genannten Beispielen können Referenzbereiche gewählt werden, die Oberflächen aufweisen, die in alle Raumrichtungen orientiert sind. Dies stellt sicher, dass z. B. in Durchstrahlungsbildern der Computertomographie Grauwertübergänge in alle Raumrichtungen vorliegen und analysiert werden können. Beispiele für Geometrien, bei denen dies besonders vorteilhaft ist, sind: eine Kugel z. B. bei einem Einschluss, eine Hohlkugel, z. B. bei einer Pore, oder quasi jede komplett geschlossene Geometrie ohne Kanten. Besonders vorteilhaft ist dies in Kombination mit dem Unterschritt: Variieren mindestens eines geometrischen Parameters der digitalen Bauteildarstellung in mindestens einer Projektion der digitalen Bauteildarstellung, Ermitteln mindestens eines Qualitätsparameters einer die mindestens eine Projektion verwendenden Rekonstruktion des Bauteils, Wiederholen des Variierens des mindestens einen geometrischen Parameters und der Ermittlung mindestens eines Qualitätsparameters bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter.

Weiter kann bei der Ermittlung der geometrischen Parameter beispielsweise weiteres Vorwissen über die Positionierung der Komponenten: Röntgenquelle, Detektor und zu messendes Bauteil zueinander berücksichtigt werden. Dies kann in einem anderen Beispiel für einzelne Projektionen bzw. Durchstrahlungsbilder berücksichtigt werden. So kann beispielsweise bekannt sein, dass sich Röntgenquelle und Detektor zum Beginn und zum Ende einer Messung in bekannten, "eingerasteten" oder eingemessenen Positionen befindet, die mit großer Genauigkeit bekannt sind, während bei anderen Projektionen die Unsicherheit der Positionierung größer ist.

Bei der Durchführung der Bestimmung der geometrischen Parameter kann auch Vorwissen über die Geometrie einfließen, wenn beispielsweise Standardgeometrien wie Gewinde oder gehornte Flächen in den Messdaten abgebildet sind, bei denen in einigen Fällen, ohne weitere Referenzmessung, davon ausgegangen werden kann, dass diese korrekt gefertigt wurden. Dieses Wissen, z. B. über die Ganghöhe des Gewindes oder dass die gehornte Fläche eine sehr gute Ebenheit aufweist, kann so einfließen, dass geometrische Parameter identifiziert werden, für die diese Vorgaben auch in den Messdaten erfüllt sind. Auf diese Weise kann zum Beispiel eine Bestimmung von zusätzlichen geometrischen Parametern durchgeführt werden, z. B. die geometrische Vergrößerung bzw. Voxelgröße. Allgemein können hierfür z. B. aus einem CAD-Modell des Bauteils abgeleitete Maße oder Geometrien verwendet werden. Dabei kann ein Katalog von Standardgeometrien erstellt werden bzw. vordefiniert sein, die in den Messdaten gesucht werden. Besonders vorteilhaft ist dieser Schritt, wenn Maße bzw. Geometrien verwendet werden, die typischerweise exakt gefertigt sind.

Gemäß einem weiteren Beispiel können Geometrien aus einer Mittelebene der digitalen Bauteildarstellung ausgewählt werden bzw. können die den Geometrien zugrundeliegenden Messdaten mit einer geringeren Unsicherheit bzw. einem entsprechenden Sicherheitsparameter versehen werden, da sich hier Kegelstrahlartefakte am geringsten auswirken.

Die Daten über die Referenzbereiche können zum Beispiel aus einer weiteren Quelle stammen, z. B. aus einer anderen Messung desselben Bauteils. Dabei kann es vorteilhaft sein, wenn innere Strukturen abgebildet werden. Beispiele hierfür sind eine hochqualitative bzw. hochauflösende computertomographische Messung oder Schliffbilder.

Falls ein CAD-Modell, eine vorläufige Rekonstruktion oder andere Informationen über die Geometrie des Bauteils vorliegt, können gemäß einem weiteren Beispiel simulierte über den gesamten Winkelraum der möglichen Durchstrahlungsrichtungen, ggf. in einer gewissen "Dichte", berechnet werden. Die vorliegenden realen Projektionen bzw. Durchstrahlungsbilder der ermittelten Messdaten können mit diesen simulierten Projektionen abgeglichen und die beste Übereinstimmung identifiziert werden. Durch diese Zuordnung kann eine grobe Aufnahmegeometrie ermittelt werden. Besonders vorteilhaft ist dieses Beispiel, wenn das Vorwissen zu der geometrischen Kalibrierung recht ungenau ist. Bei Bedarf können in einem weiteren Beispiel zusätzlich gezielt weitere relevante simulierte Projektionen nachberechnet werden, wenn lokal die "Dichte" der Durchstrahlungsrichtungen für die Ermittlung der Aufnahmegeometrie nicht ausreicht.

Bei der Durchführung des erfindungsgemäßen Verfahrens kann ein einem anderen Beispiel, das eine computertomographischen Messung verwendet, berücksichtigt werden, dass es eine Spektrum-abhängige Eindringtiefe der Photonen in den Detektor gibt. Bauteile unterschiedlicher Materialien können somit bei einer Kegelstrahl-Geometrie in unterschiedlichen effektiven Vergrößerungen abgebildet sein, was Messfehler verursacht. Eine Berücksichtigung dieses Effekts kann diese Messfehler minimieren.

Falls Geometrien in den Projektionen identifiziert werden, kann es in einem weiteren Beispiel vorteilhaft sein, diese für jede Projektion separat zu identifizieren.

Wenn das Bauteil mittels einer additiven Fertigung hergestellt wurde oder wird, kann es beispielsweise vorteilhaft sein, geeignete Geometrien, z. B. kleine kugelförmige Strukturen, im Inneren des Objekts zu platzieren, damit diese für das erfindungsgemäße Verfahren verwendet werden können.

Ein weiterer Aspekt, außerhalb der beanspruchten Erfindung, betrifft ein Computerprogrammprodukt mit auf einem Computer ausführbaren Instruktionen, welche auf einem Computer ausgeführt den Computer dazu veranlassen, das Verfahren nach der vorhergehenden Beschreibung durchzuführen. Vorteile und Wirkungen sowie Weiterbildungen des Computerprogrammprodukts ergeben sich aus den Vorteilen und Wirkungen sowie Weiterbildungen des oben beschriebenen Verfahrens. Es wird daher in dieser Hinsicht auf die vorangegangene Beschreibung verwiesen.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigt:

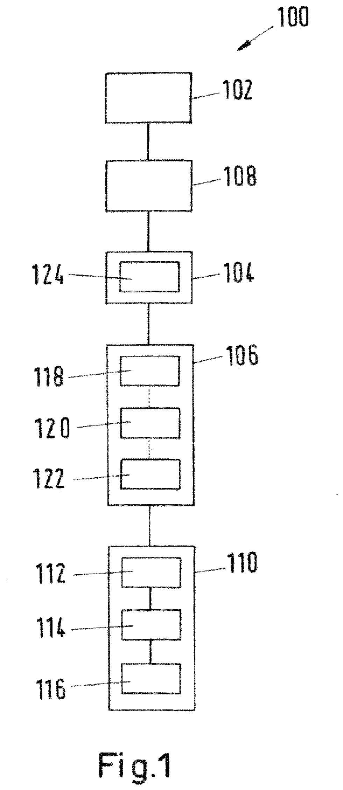

- Fig. 1: ein Flussdiagramm des computerimplementierten Verfahrens zur Ermittlung mindestens eines für eine Auswertung von Messdaten benötigten geometrischen Parameters.

Das computerimplementierten Verfahren zur Ermittlung mindestens eines für eine Auswertung von Messdaten benötigten geometrischen Parameters wird im Folgenden gemäß Figur 1 in seiner Gesamtheit mit dem Bezugszeichen 100 bezeichnet.

Mit dem Verfahren 100 werden geometrische Parameter ermittelt, mit denen Messdaten aus einer durchstrahlenden Messung eines Bauteils ausgewertet werden. Der mindestens eine geometrische Parameter kann z. B. eine für die durchstrahlende Messung des Bauteils verwendete Aufnahmegeometrie beschreiben.

In einem ersten Schritt 102 werden die Messdaten ermittelt. Die Messdaten können dabei z. B. aus einer computertomographischen Messung erhalten werden und Projektionen bzw. Durchstrahlungsbilder des Bauteils sein, das eine Bauteilgeometrie aufweist. Die Bauteilgeometrie beschreibt die reale dreidimensionale Form des Bauteils. Aus den Messdaten kann eine digitale Bauteildarstellung erzeugt werden, z. B. über eine Rekonstruktion der Volumendaten des Bauteils aus den Projektionen.

In einem optionalen Schritt 108 kann zumindest aus einem Teil der ermittelten Messdaten eine vorläufige Rekonstruktion durchgeführt werden, um vorläufige Volumendaten zu erhalten. Weiter wird damit eine vorläufige digitale Bauteildarstellung erhalten. Die vorläufige Rekonstruktion wird mittels mindestens eines vordefinierten geometrischen Start-Parameter durchgeführt. Der vordefinierte geometrische Start-Parameter kann durch einen Benutzer oder ein alternatives Verfahren vordefiniert werden oder ein Schätz- bzw. Erfahrungswert sein, der aus früheren Verwendungen des Verfahrens 100 als damals finaler geometrischer Parameter für gleiche oder ähnliche Bauteile ermittelt wurde.

Danach wird in einem Schritt 104 mindestens ein Referenzbereich in der digitalen Bauteildarstellung oder in der Bauteilgeometrie identifiziert, z. B. Bereiche, in denen Material- und/oder Luft-Einschlüsse angeordnet sind, und/oder Bereiche, die eine Geometrie aufweisen, die kugelförmig ist oder unter Berücksichtigung eines vordefinierten Toleranzbereiches als kugelförmig klassifiziert wird. Weiter können z. B. Bereiche identifiziert werden, die ein Objekt einer Baugruppe im Bauteil sind und einen Absorptionskoeffizienten aufweisen, der innerhalb eines vordefinierten Intervalls angeordnet ist, oder die in einem Volumen der Bauteilgeometrie angeordnet sind, in dem keine Referenzbereiche oder eine Anzahl an Referenzbereichen, die kleiner als ein vordefinierter Grenzwert ist, identifiziert wurden. Der mindestens eine Referenzbereich umfasst dabei lediglich einen Teil der digitalen Bauteildarstellung bzw. der Bauteilgeometrie.

Der Schritt 104 kann den optionalen Unterschritt 124 aufweisen, mit dem Grenzflächen in der digitalen Bauteildarstellung segmentiert werden.

Wenn der Schritt 108 durchgeführt wurde, kann die Identifizierung des Referenzbereichs in der digitalen Bauteildarstellung, die mittels der vorläufigen Rekonstruktion erhalten wurde, durchgeführt werden. Alternativ oder zusätzlich kann die Identifizierung in einer Segmentierung der digitalen Bauteildarstellung durchgeführt werden.

Referenzbereiche und ihre Positionen werden anhand einer Messung ermittelt, in der sich die bisherigen Fehler bzw. Ungenauigkeiten der für die Rekonstruktion relevanten geometrischen Parameter noch auswirken. Somit könnten sich diese Fehler teilweise in den Referenzbereichen bzw. in deren Position wiederspiegeln und somit nicht in den Projektionen identifizierbar bzw. korrigierbar sein. Um einen Volumenbereich zu rekonstruieren werden Daten von unterschiedlichen Bereichen des Detektors verwendet, so dass sich lokale Abweichungen rausmitteln können. Daher hat die Rekonstruktion der Volumendaten, auf deren Basis die Referenzbereiche ermittelt werden, eine ausgleichende Wirkung auf die Fehler. Daher wirken sich diese Fehler tendenziell wenig auf die Referenzbereiche bzw. deren Position aus. Analog kann auch der Schritt einer Segmentierung bzw. Oberflächenbestimmung eine ausgleichende Wirkung haben. Auf Basis der Informationen der Referenzbereiche können diese Fehler ermittelt und bei der nächsten Rekonstruktion korrigiert werden.

Wenn zum Beispiel ein unbekannter seitlicher Versatz des Detektors um wenige Pixel vorliegt, verursacht dies bei einer axialen Computertomographie in erster Näherung keine systematischen Verzerrungen der Volumendaten. Es wird lediglich eine Unschärfe innerhalb einer x-y-Ebene des Detektors verursacht. Die (Schwerpunkts-)Positionen der Referenzbereiche, z. B. Defekte, bleiben hiervon unbeeinflusst, so dass der Referenzbereich "korrekt" ist und für eine Korrektur verwendet werden kann.

Referenzbereiche können, z. B. mittels eines CAD-Modells des Bauteils identifiziert werden. Das CAD-Modell ist ein Beispiel für Vorwissen über das zu messende Bauteil. Die in dem CAD-Modell festgelegte Bauteilgeometrie wird dann verwendet, um passende Bereiche des Bauteils auszuwählen, die als Referenzbereiche geeignet sind, um damit geometrische Parameter für die Auswertung der Messdaten zu gewinnen. Dies können z. B. Bereiche sein, die in der durchstrahlenden Messung sicher und mit hoher Qualität abgebildet werden können. Dies können z. B. Bereiche sein, von denen erwartet wird, dass sie aufgrund ihrer Geometrie oder ihrer Materialzusammensetzung deutlich in den Messdaten sichtbar sind.

Alternativ oder zusätzlich können die Bereiche aus der digitalen Bauteildarstellung bzw. deren Segmentierung ausgewählt werden, ohne Vorwissen zu verwenden. Dazu können z. B. in den Durchstrahlungsbildern zunächst Bereiche mit hohem Kontrast und/oder scharfen Kanten als Referenzbereiche ausgewählt werden. Die Durchstrahlungsbilder können dazu einzeln auf passende Bereiche ausgewertet werden. Von diesen Bereichen werden die Bereiche ausgewählt, die in der digitalen Bauteildarstellung und/oder deren Segmentierung abgebildet werden.

Das Identifizieren von Bereichen in der digitalen Bauteildarstellung kann zum Beispiel bedeuten, dass ohne spezielles Vorwissen über die Geometrie des Bauteils Referenzbereiche gesucht werden, die besonders gut für die geometrische Kalibrierung geeignet sind. Weiter kann das Identifizieren zum Beispiel bedeuten, dass die Position von bekannten bzw. definierten Referenzbereiche des Bauteils in den Messdaten ermittelt wird.

Mittels der Referenzbereiche wird in Schritt 106 mindestens ein geometrischer Parameter für die Auswertung der Messdaten ermittelt. Dabei kann z. B. ein Sicherheitsparameter der Referenzbereiche berücksichtigt werden. Die Auswertung kann z. B. eine finale Rekonstruktion sein, d. h. die Erzeugung von Volumendaten aus den Messdaten. Alternativ kann die Auswertung eine Messung von Geometrien direkt in den Projektionsdaten sein, wobei die geometrische Zuordnung der Projektionen zueinander berücksichtigt wird.

Die geometrischen Parameter können zum Beispiel die Aufnahmegeometrie, die Abweichungen des Detektors von der Nominalgestalt oder ein nicht-rigides Verhalten, z. B. Verformungen, des Bauteils während der Messung beschreiben.

Abweichungen des Detektors von der Nominalgestalt können z. B. Verformungen des Detektors sein, insbesondere in der Roboter-Computertomographie. Weiter können die Abweichungen aus baulichen Toleranzen des Detektors resultieren, wenn z. B. Pixelabstände gewisse Abweichungen aufweisen.

Die Unterschritte 118, 120 und 122 des Schritts 106 beschreiben im Folgenden miteinander optional kombinierbare Verfahrensschritte zur Ermittlung mindestens eines geometrischen Parameters.

Im Unterschritt 118 kann dazu die Positionen der Referenzbereiche zueinander in der digitalen Bauteildarstellung ermittelt werden. Dies kann sowohl die Unsicherheit der Bestimmung bzw. Lokalisierung der Geometrie in drei Dimensionen umfassen als auch die Unsicherheit, mit der eine Geometrie in einer zweidimensionalen Projektion erkannt bzw. ihre Position und/oder Ausrichtung ermittelt werden kann. Weiter werden in diesem Unterschritt projizierte Positionen der Referenzbereiche in mindestens einem Teil der ermittelten Messdaten ermittelt und diese dazu verwendet, um den mindestens einen geometrischen Parameter zu ermitteln.

Optional können in dem Unterschritt 118 Geometrien der Referenzbereiche beim Ermitteln der projizierten Positionen berücksichtigt werden.

Mit dem Unterschritt 120 kann eine durchstrahlende Messung an der digitalen Bauteildarstellung und/oder einer auf der digitalen Bauteildarstellung basierenden Segmentierung simuliert werden. Die Simulation erzeugt simulierte Messdaten, in denen die Referenzbereiche ebenfalls abgebildet sind. Die Referenzbereiche der simulierten Messdaten werden mit den Referenzbereichen der ermittelten Messdaten verglichen. Bereiche der Volumendaten und/oder Messdaten bzw. Projektionen, die z. B. ein erhöhtes Rauschen aufweisen, werden bei der Beurteilung der Übereinstimmung von simulierten und ermittelten Messdaten weniger berücksichtigt, da der Informationsgehalt dieser Bereiche geringer ist. Das Ergebnis des Vergleichs wird verwendet, um den mindestens einen geometrischen Parameter zu ermitteln.

Optional können im Unterschritt 120 die simulierten Messdaten lediglich für Bereiche in der digitalen Bauteildarstellung erzeugt werden, die Referenzbereiche aufweisen.

Weiter kann mit dem Unterschritt 122 mindestens ein geometrischer Parameter der digitalen Bauteildarstellung in mindestens einer Projektion bzw. einem Durchstrahlungsbild der digitalen Bauteildarstellung variiert werden. Mit dem mindestens einen variierten geometrischen Parameter wird eine Rekonstruktion des Bauteils durchgeführt, die zumindest auf der mindestens einen Projektion basiert. Für diese Rekonstruktion wird mindestens ein Qualitätsparameter in mindestens einem Referenzbereich ermittelt. Das Variieren des mindestens einen geometrischen Parameters und die Ermittlung des mindestens eines Qualitätsparameters in dem mindestens einen Referenzbereich wird wiederholt, bis mindestens ein optimierter geometrischer Parameter einen Qualitätsparameter aufweist, der ein Maximalwert aller ermittelten Qualitätsparameter ist. Dabei wird durch das Variieren des mindestens einen geometrischen Parameters eine variierte Rekonstruktion geschaffen, deren Qualität erneut bewertet wird. Bereiche der Volumendaten und/oder Messdaten bzw. Projektionen, die z. B. ein erhöhtes Rauschen aufweisen, werden bei der Beurteilung der Qualität der Volumendaten weniger berücksichtigt, da der Informationsgehalt dieser Bereiche geringer ist. Wenn ein gemäß dem oben genannten Abbruchkriterium ein optimierter geometrischer Parameter ermittelt wurde, wird dieser als der mindestens eine geometrische Parameter für die Auswertung der Messdaten verwendet.

Optional können im Unterschritt 122 lediglich die Bereiche der digitalen Bauteildarstellung mittels der variierten geometrischen Parameter in der digitalen Bauteildarstellung neu rekonstruiert und analysiert werden, die Referenzbereiche aufweisen.

Mit Hilfe der Referenzbereiche können Informationen über, ggf. lokale, Abweichungen des Detektors von der Nominalgestalt, d. h. einer idealen Detektorgeometrie, abgeleitet werden. Insbesondere, falls sehr viele hochqualitative Referenzbereiche vorliegen und die geometrische Kalibrierung bekannt ist.

Ein realer Detektor ist nicht perfekt, da der Abstand zwischen den Pixeln in der Regel nicht konstant ist, außerdem können Verformungen senkrecht zur Detektorfläche auftreten. Es gibt somit Abweichungen des Detektors von einer perfekten Pixelmatrix. Diese Abweichungen, quasi drei Freiheitsgrade pro Pixel, sind in erster Näherung konstant über viele Messungen. Allerdings können sie zwischen verschiedenen Projektionen auch variieren, z. B. bei einem Detektor, der von einem Roboter-Arm positioniert wird und sich unter dem Eigengewicht oder durch thermale Effekte in verschiedenen Orientierungen unterschiedlich verformt. Beschrieben werden können diese Abweichungen beispielsweise durch eine pixelweise Definition der Abweichung des jeweiligen Pixels von der Nominal-Position. Diese Abweichungen von einer idealen Detektorgeometrie können anhand einer Analyse einer Projektion üblicherweise nicht identifiziert werden, so lange die durchstrahlte bzw. abgebildete Geometrie nicht bekannt sind. Dadurch, dass die Referenzbereiche und ihre Positionen zueinander bekannt sind, können gewisse, ggf. lokale, Abweichungen mit Hilfe der Projektionen ermittelt werden, so dass sie daraufhin korrigiert bzw. in der Rekonstruktion berücksichtigt werden können. Es kann auch eine z-Komponente geben, z. B. wenn der Detektor verbogen ist. Die Verzerrungen können abhängig von der Temperatur und somit für jede Projektion unterschiedlich sein. Dennoch sind zumeist die größten Anteile über die Projektionen konstant. Vorteilhafterweise werden dann für jeden Pixel globale Verzerrungen, z. B. in Form von Abweichungsvektoren, ermittelt.

Um die Anzahl der Freiheitsgrade zu verringern kann es vorteilhaft sein, nur für einen Teil der Pixel die Abweichungsvektoren zu ermitteln und dazwischen zu interpolieren.

Mit Hilfe der Referenzbereiche werden geometrische Parameter zu einem nicht-rigiden Verhalten, z. B. Verformungen, des Bauteils während der Messung bzw. für jede Projektion ermittelt, insbesondere, falls sehr viele hochqualitative Referenzbereiche vorliegen und die geometrische Kalibrierung bekannt ist.

Ohne Korrektur sind die Messdaten bei einer Verformung des gemessenen Bauteils inkonsistent. Dies äußert sich durch Bildfehler in den Volumendaten. Das nicht-rigide Verhalten kann in der Rekonstruktion korrigiert bzw. berücksichtigt werden, so dass ein konsistentes und möglichst fehlerfreies Volumen rekonstruiert werden kann.

Das nicht-rigide Verhalten des Bauteils kann bspw. durch ein Feld von Verschiebungsvektoren dargestellt werden. Hierbei sind verschiedene Auflösungen des Felds und hieraus resultierend eine entsprechend variierende Gesamtzahl von Stützpunkten möglich. Da die Anzahl der Freiheitsgrade wie auch bei den Abweichungen des Detektors von der Nominalgestalt vergleichsweise groß ist, werden für eine Bestimmung der geometrischen Parameter vergleichsweise viele Referenzbereiche benötigt.

Anwendungsbeispiele sind z. B. dünnwandige Bauteile, welche sich unter ihrem eigenen Gewicht während der Messung verformen, oder durch thermische Effekte ausgelöste Verformungen, z. B. Erwärmung des Bauteils in begrenzten Regionen.

Vorteilhafterweise liegt anfangs mindestens eine grobe Abschätzung der gesuchten geometrischen Parameter vor. Es ist jedoch ausreichend, wenn die grobe Aufnahmegeometrie nur für einzelne Projektionen bekannt ist, da dies z. B. für eine vorläufige Rekonstruktion ausreicht. Weiterhin kann mit anderen Verfahren eine erste grobe Abschätzung der Parameter durchgeführt werden, beispielsweise unter Nutzung von Konsistenzbedingungen bezüglich Linienintegralen in unterschiedlichen Projektionen bzw. den sogenannten Epipolar Consistency Conditions, woraufhin mit Hilfe der Erfindung die genaue Bestimmung durchgeführt wird.

Bevorzugt wird die Erfindung bei Messungen eingesetzt, die einen Flächendetektor verwenden.

Für eine exakte Lokalisierung erkannter Geometrien des Bauteils kann jeweils eine klar definierte Position der Geometrie verwendet werden, z. B. der Massenschwerpunkt oder ein Schnittpunkt von Kanten wie die Ecke eines Würfels.

Mit weiteren Verfahrensschritten können weiter die zwei Winkel der Detektorverkippung, die "out-of-plane" angeordnet sind, d. h. die beiden Richtungen, die nicht die Detektor-Normale als Drehachse haben, ermitteln werden. Damit kann die Ermittlung der geometrischen Vergrößerung bzw. Voxelgröße, der out-of-plane-Verkippungen des Detektors, und/oder weiterer geometrischer Parameter durchgeführt werden. Dazu kann beispielsweise die relative Positionierung von Detektor, Röntgenquelle und Drehachse mit Hilfe von Sensoren ermittelt werden, z. B. mittels eines Laserinterferometers.

In einem anderen Beispiel kann sich während der Messung ein weiteres Objekt bekannter Geometrie, z. B. zwei Kugeln in einem bekannten Abstand zueinander, im Messbereich befinden. In einem weiteren Beispiel kann das Wissen über gewisse Geometrien des Bauteils genutzt werden, beispielsweise wenn eine Referenzmessung über einen Teil der Geometrien des Bauteils vorliegt, um dieses Wissen auf die gesamte Messung zu übertragen.

Gemäß einem Beispiel für den Anwendungsfall einer geometrischen Kalibrierung eines axialen Computertomographen oder eines Spiral- Computertomographen können zunächst die globalen Parameter bestimmt werden. Es wird somit zunächst die Anzahl der Freiheitsgrade sinnvoll beschränkt. Dies wird als Startwert bzw. Ausgangspunkt für eine Bestimmung der lokalen geometrischen Parameter bzw. der Abweichungen der einzelnen Projektionen von der idealen Trajektorie in dem axialen Computertomographen bzw. Spiral- Computertomographen verwendet.

Nach dem Schritt 106 kann das Verfahren den optionalen Schritt 110 aufweisen. Im Schritt 110 werden optionale Unterschritte 112, 114 und 116 wiederholt, solange ein vordefiniertes Abbruchkriterium nicht erfüllt ist.

Im Unterschritt 112 wird eine weitere vorläufige Rekonstruktion des Bauteils als digitale Bauteildarstellung ermittelt. Dazu wird zumindest ein Teil der ermittelten Messdaten verwendet. Die Ermittlung der Messdaten muss dabei nicht zwingend abgeschlossen worden sein und kann parallel weiter durchgeführt werden. Die bereits vorhandenen Messdaten oder ein Teil der vorhandenen Messdaten können dabei den zumindest einen Teil der ermittelten Messdaten bilden. Alternativ kann nach dem Ende der Ermittlung der Messdaten ein Teil der bereits vorhandenen Messdaten für die vorläufige Rekonstruktion verwendet werden. Die Rekonstruktion wird mittels des in Schritt 106 ermittelten geometrischen Parameters durchgeführt.

Es werden danach im Unterschritt 114 erneut Bereiche in der digitalen Bauteildarstellung als Referenzbereiche identifiziert. Da die digitale Bauteildarstellung mittels des geometrischen Parameters ermittelt wurde, ist die im Unterschritt 114 verwendete digitale Bauteildarstellung tendenziell genauer als die in Schritt 104. D. h. auch die im Unterschritt 114 ermittelten Referenzbereiche haben tendenziell eine bessere Qualität als die in Schritt 104 ermittelten.

Im Unterschritt 116 wird mittels der Referenzbereiche aus Unterschritt 114 mindestens ein weiterer geometrischer Parameter ermittelt, der den vorherigen mindestens einen geometrischen Parameter ersetzt. Wegen der tendenziell besseren Referenzbereiche ist der mindestens eine weitere geometrische Parameter tendenziell genauer als der im Schritt 106 ermittelte geometrische Parameter.

Bei der Wiederholung der Unterschritte 112 bis 116 wird der im Unterschritt 114 ermittelte mindestens eine weitere geometrische Parameter als der mindestens eine geometrische Parameter behandelt.

Das vordefinierte Abbruchkriterium kann z. B die Bedingung umfassen, dass der Unterschied zwischen dem neu ermittelten mindestens einen geometrischen Parameter und dem vorherigen geometrischen Parameter unterhalb eines vordefinierten Schwellenwerts liegt und damit der neu ermittelte mindestens eine geometrische Parameter keine wesentliche Verbesserung gegenüber dem vorherigen mindestens einem geometrischen Parameter darstellt.

Die in diesem Beispiel beschriebene Reihenfolge der Schritte kann beliebig, soweit vernünftig ausführbar, verändert werden.

Das computerimplementierte Verfahren 100 kann mittels eines Computerprogrammprodukts auf einem Computer ausgeführt werden. Das Computerprogrammprodukt weist dabei auf einem Computer ausführbaren Instruktionen auf. Wenn diese Instruktionen auf einem Computer ausgeführt werden, veranlassen sie den Computer dazu, das Verfahren durchzuführen.

Die Erfindung ist nicht auf eine der vorbeschriebenen Ausführungsformen beschränkt, sondern in vielfältiger Weise abwandelbar. Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Computerimplementiertes Verfahren zur Ermittlung mindestens eines für eine Auswertung von Messdaten benötigten geometrischen Parameters, wobei die Messdaten mittels einer durchstrahlenden Messung eines Bauteils, das eine Bauteilgeometrie aufweist, ermittelt wurden, wobei durch die Messdaten eine digitale Bauteildarstellung erzeugt wird, wobei das Verfahren (100) die folgenden Schritte aufweist:
- Erhalten (102) der mittels einer durchstrahlenden Messung eines Bauteils ermittelten Messdaten;
- Identifizieren (104) von Bereichen in der digitalen Bauteildarstellung und/oder in der Bauteilgeometrie als Referenzbereiche;
- Ermitteln (106) mindestens eines geometrischen Parameters, der für eine Auswertung der ermittelten Messdaten benötigt wird, mittels der Referenzbereiche,
wobei ein Referenzbereich lediglich einen Teil des Bauteils umfasst und das Verfahren (100) als Online-Kalibrierung durchgeführt wird, und
wobei der Schritt (106): Ermitteln mindestens eines geometrischen Parameters, mindestens einen der folgenden Unterschritte (118, 120, 122) aufweist:
- Ermitteln (118) der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters; und/oder
- Simulieren (120) einer durchstrahlenden Messung an der digitalen Bauteildarstellung und/oder einer auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters; und/oder
- Variieren (122) mindestens eines geometrischen Parameters der digitalen Bauteildarstellung in mindestens einer Projektion der digitalen Bauteildarstellung, Ermitteln mindestens eines Qualitätsparameters in mindestens einem Referenzbereich einer die mindestens eine Projektion verwendenden Rekonstruktion des Bauteils, Wiederholen des Variierens des mindestens einen geometrischen Parameters und der Ermittlung mindestens eines Qualitätsparameters in mindestens einen Referenzbereich bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter,
**dadurch gekennzeichnet, dass** im Schritt (104) für jeden Referenzbereich ermittelt wird, welcher Unterschritt (118, 120, 122) zur Ermittlung der geometrischen Parameter mittels des Referenzbereichs eingesetzt werden kann.

2. Verfahren nach Anspruch 1, **dadurch gekennzeichnet, dass** der mindestens eine geometrische Parameter eine für die durchstrahlende Messung des Bauteils verwendete Aufnahmegeometrie beschreibt.

3. Verfahren nach Anspruch 1 oder 2, **dadurch gekennzeichnet, dass** das Verfahren (100) zwischen dem Schritt (102): Ermitteln der Messdaten, und dem Schritt (104): Identifizieren von Bereichen in der digitalen Bauteildarstellung und/oder in der Bauteilgeometrie als Referenzbereiche, folgenden Schritt aufweist:
- Ermitteln (108) einer vorläufigen Rekonstruktion des Bauteils als digitale Bauteildarstellung aus zumindest einem Teil der ermittelten Messdaten mittels mindestens eines vordefinierten geometrischen Start-Parameters,
wobei im Schritt (104): Identifizieren von Bereichen in der digitalen Bauteildarstellung und/oder in der Bauteilgeometrie als Referenzbereiche, die Bereiche in der digitalen Bauteildarstellung und/oder in einer auf der digitalen Bauteildarstellung basierenden Segmentierung identifiziert werden.

4. Verfahren nach Anspruch 3, **dadurch gekennzeichnet, dass** das Verfahren (100) nach dem Schritt (106): Ermitteln mindestens eines geometrischen Parameters, weiter folgende Schritte aufweist:
- Wiederholen (110) der folgenden Schritte solange ein vordefiniertes Abbruchkriterium nicht erfüllt wird:
- Ermitteln (112) einer weiteren vorläufigen Rekonstruktion des Bauteils als digitale Bauteildarstellung aus zumindest einem Teil der ermittelten Messdaten mittels des mindestens einen geometrischen Parameters;
- Identifizieren (114) von Bereichen in der digitalen Bauteildarstellung als Referenzbereiche;
- Ermitteln (116) mindestens eines weiteren geometrischen Parameters, der den mindestens einen geometrischen Parameter ersetzt und für eine Auswertung der Messdaten benötigt wird, mittels der Referenzbereiche.

5. Verfahren nach einem der Ansprüche 1 bis 4, **dadurch gekennzeichnet, dass** im Unterschritt (118): Ermitteln der Positionen der Referenzbereiche zueinander in der digitalen Bauteildarstellung, Ermitteln von projizierten Positionen der Referenzbereiche in mindestens einem Teil der ermittelten Messdaten und Verwenden der Positionen der Referenzbereiche und der projizierten Positionen der Referenzbereiche zum Ermitteln mindestens eines geometrischen Parameters; Geometrien der Referenzbereiche beim Ermitteln der projizierten Positionen berücksichtigt werden.

6. Verfahren nach einem der Ansprüche 1 bis 4, **dadurch gekennzeichnet, dass** im Unterschritt (120): Simulieren einer durchstrahlenden Messung an der digitalen Bauteildarstellung und/oder der auf der digitalen Bauteildarstellung basierenden Segmentierung, wobei die Simulation simulierte Messdaten erzeugt, und Verwenden eines Vergleichs der Referenzbereiche in den ermittelten Messdaten und den simulierten Messdaten zum Ermitteln mindestens eines geometrischen Parameters; die simulierten Messdaten lediglich für Bereiche in der digitalen Bauteildarstellung erzeugt werden, die Referenzbereiche aufweisen.

7. Verfahren nach einem der Ansprüche 1 bis 4, **dadurch gekennzeichnet, dass** im Unterschritt (122): Variieren mindestens eines geometrischen Parameters der digitalen Bauteildarstellung in mindestens einer Projektion der digitalen Bauteildarstellung, Ermitteln mindestens eines Qualitätsparameters in mindestens einem Referenzbereich einer die mindestens eine Projektion verwendenden Rekonstruktion des Bauteils, Wiederholen des Variierens des mindestens einen geometrischen Parameters und der Ermittlung mindestens eines Qualitätsparameters in mindestens einen Referenzbereich bis mindestens ein optimierter geometrischer Parameter einen Qualitätsparameter aufweist, der ein Maximalwert aller ermittelten Qualitätsparameter ist, und Verwenden des mindestens einen optimierten geometrischen Parameters als ermittelten mindestens einen geometrischen Parameter; lediglich die Bereiche der digitalen Bauteildarstellung mittels der variierten geometrischen Parameter in der digitalen Bauteildarstellung neu rekonstruiert und analysiert werden, die Referenzbereiche aufweisen.

8. Verfahren nach einem der Ansprüche 1 bis 7, **dadurch gekennzeichnet, dass** der Schritt (104): Identifizieren von Bereichen in der digitalen Bauteildarstellung und/oder in der Bauteilgeometrie als Referenzbereiche, den folgenden Unterschritt aufweist:
- Segmentieren (124) von Grenzflächen der digitalen Bauteildarstellung.

9. Verfahren nach einem der Ansprüche 1 bis 8, **dadurch gekennzeichnet, dass** im Schritt (104): Identifizieren von Bereichen in der digitalen Bauteildarstellung und/oder in der Bauteilgeometrie als Referenzbereiche, Bereiche identifiziert werden, in denen Material- und/oder Luft-Einschlüsse angeordnet sind.

10. Verfahren nach einem der Ansprüche 1 bis 9, **dadurch gekennzeichnet, dass** im Schritt (104): Identifizieren von Bereichen in der digitalen Bauteildarstellung und/oder in der Bauteilgeometrie als Referenzbereiche, Bereiche identifiziert werden, die eine Geometrie aufweisen, die kugelförmig ist oder unter Berücksichtigung eines vordefinierten Toleranzbereiches als kugelförmig klassifiziert wird.

11. Verfahren nach einem der Ansprüche 1 bis 10, **dadurch gekennzeichnet, dass** im Schritt (104): Identifizieren von Bereichen in der digitalen Bauteildarstellung und/oder in der Bauteilgeometrie als Referenzbereiche, Bereiche identifiziert werden, die ein Objekt einer Baugruppe im Bauteil sind und einen Absorptionskoeffizienten aufweisen, der innerhalb eines vordefinierten Intervalls angeordnet ist.

12. Verfahren nach einem der Ansprüche 1 bis 11, **dadurch gekennzeichnet, dass** im Schritt (104): Identifizieren von Bereichen in der digitalen Bauteildarstellung und/oder in der Bauteilgeometrie als Referenzbereiche, Bereiche identifiziert werden, die in einem Volumen der Bauteilgeometrie angeordnet sind, in dem keine Referenzbereiche oder eine Anzahl an Referenzbereichen, die kleiner als ein vordefinierter Grenzwert ist, identifiziert wurden.

13. Verfahren nach einem der Ansprüche 1 bis 12, **dadurch gekennzeichnet, dass** im Schritt (106): Ermitteln mindestens eines geometrischen Parameters, der für eine Auswertung der Messdaten benötigt wird, mittels der Referenzbereiche, Sicherheitsparameter der Referenzbereiche berücksichtigt werden, wobei ein Sicherheitsparameter angibt, wie sicher eine Position des ihm zugeordneten Referenzbereichs ist.

## Claims

1. Computer-implemented method for determining at least one geometric parameter required for an evaluation of measurement data, wherein the measurement data were determined by means of a radiographic measurement of a component having a component geometry, wherein a digital component representation is generated by the measurement data, wherein the method (100) comprises the following steps:
- obtaining (102) the measurement data determined by means of a radiographic measurement of a component;
- identifying (104) regions in the digital component representation and/or in the component geometry as reference regions;
- determining (106) at least one geometric parameter required for an evaluation of the determined measurement data, by means of the reference regions,
wherein a reference region comprises only a portion of the component and the method (100) is carried out as online calibration, and
wherein step (106): determining at least one geometric parameter comprises at least one of the following substeps (118, 120, 122):
- determining (118) the positions of the reference regions with respect to one another in the digital component representation, determining projected positions of the reference regions in at least one portion of the determined measurement data, and using the positions of the reference regions and the projected positions of the reference regions for determining at least one geometric parameter; and/or
- simulating (120) a radiographic measurement on the digital component representation and/or a segmentation based on the digital component representation, wherein the simulation generates simulated measurement data, and using a comparison of the reference regions in the determined measurement data and the simulated measurement data for determining at least one geometric parameter; and/or
- varying (122) at least one geometric parameter of the digital component representation in at least one projection of the digital component representation, determining at least one quality parameter in at least one reference region of a reconstruction of the component that uses the at least one projection, repeating the processes of varying the at least one geometric parameter and determining at least one quality parameter in at least one reference region until at least one optimized geometric parameter has a quality parameter that is a maximum value of all determined quality parameters, and using the at least one optimized geometric parameter as determined at least one geometric parameter,
**characterized in that** step (104) involves determining, for each reference region, which substep (118, 120, 122) can be used for determining the geometric parameters by means of the reference region.

2. Method according to Claim 1, **characterized in that** the at least one geometric parameter describes a recording geometry used for the radiographic measurement of the component.

3. Method according to Claim 1 or 2, **characterized in that** between step (102): determining the measurement data, and step (104): identifying regions in the digital component representation and/or in the component geometry as reference regions, the method (100) comprises the following step:
- determining (108) a provisional reconstruction of the component as digital component representation from at least one portion of the determined measurement data by means of at least one predefined geometric start parameter,
wherein in step (104): identifying regions in the digital component representation and/or in the component geometry as reference regions, the regions are identified in the digital component representation and/or in a segmentation based on the digital component representation.

4. Method according to Claim 3, **characterized in that** after step (106): determining at least one geometric parameter, the method (100) furthermore comprises the following steps:
- repeating (110) the following steps as long as a predefined termination criterion is not satisfied:
- determining (112) a further provisional reconstruction of the component as digital component representation from at least one portion of the determined measurement data by means of the at least one geometric parameter;
- identifying (114) regions in the digital component representation as reference regions;
- determining (116) at least one further geometric parameter that replaces the at least one geometric parameter and is required for an evaluation of the measurement data, by means of the reference regions.

5. Method according to any of Claims 1 to 4, **characterized in that** in substep (118):
determining the positions of the reference regions with respect to one another in the digital component representation, determining projected positions of the reference regions in at least one portion of the determined measurement data, and using the positions of the reference regions and the projected positions of the reference regions for determining at least one geometric parameter; geometries of the reference regions are taken into account when determining the projected positions.

6. Method according to any of Claims 1 to 4, **characterized in that** in substep (120): simulating a radiographic measurement on the digital component representation and/or the segmentation based on the digital component representation, wherein the simulation generates simulated measurement data, and using a comparison of the reference regions in the determined measurement data and the simulated measurement data for determining at least one geometric parameter; the simulated measurement data are generated only for regions in the digital component representation which have reference regions.

7. Method according to any of Claims 1 to 4, **characterized in that** in substep (122): varying at least one geometric parameter of the digital component representation in at least one projection of the digital component representation, determining at least one quality parameter in at least one reference region of a reconstruction of the component that uses the at least one projection, repeating the process of varying the at least one geometric parameter and determining at least one quality parameter in at least one reference region until at least one optimized geometric parameter has a quality parameter that is a maximum value of all determined quality parameters, and using the at least one optimized geometric parameter as determined at least one geometric parameter; only the regions of the digital component representation which have reference regions are newly reconstructed and analysed by means of the varied geometric parameters in the digital component representation.

8. Method according to any of Claims 1 to 7, **characterized in that** step (104): identifying regions in the digital component representation and/or in the component geometry as reference regions comprises the following substep:
- segmenting (124) interfaces of the digital component representation.

9. Method according to any of Claims 1 to 8, **characterized in that** in step (104): identifying regions in the digital component representation and/or in the component geometry as reference regions, regions in which material and/or air inclusions are arranged are identified.

10. Method according to any of Claims 1 to 9, **characterized in that** in step (104): identifying regions in the digital component representation and/or in the component geometry as reference regions, regions are identified which have a geometry which is spherical or is classified as spherical taking account of a predefined tolerance range.

11. Method according to any of Claims 1 to 10, **characterized in that** in step (104): identifying regions in the digital component representation and/or in the component geometry as reference regions, regions are identified which are an object of an assembly in the component and have an absorption coefficient which is arranged within a predefined interval.

12. Method according to any of Claims 1 to 11, **characterized in that** in step (104): identifying regions in the digital component representation and/or in the component geometry as reference regions, regions are identified which are arranged in a volume of the component geometry in which no reference regions or a number of reference regions which is less than a predefined limit value were identified.

13. Method according to any of Claims 1 to 12, **characterized in that** in step (106): determining at least one geometric parameter required for an evaluation of the measurement data, by means of the reference regions, certainty parameters of the reference regions are taken into account, a certainty parameter indicating the level of certainty of a position of the reference region assigned to it.

## Revendications

1. Procédé informatisé de détermination d'au moins un paramètre géométrique nécessaire à l'évaluation de données de mesure, les données de mesure ayant été déterminées au moyen d'une mesure en transmission d'un composant qui présente une géométrie de composant, une représentation de composant numérique étant générée à partir des données de mesure, le procédé (100) comportant les étapes suivantes :
- obtenir (102) les données de mesure déterminées au moyen d'une mesure en transmission d'un composant ;
- identifier (104) des zones dans la représentation de composant numérique et/ou dans la géométrie de composant comme zones de référence ;
- déterminer (106) au moyen des zones de référence au moins un paramètre géométrique qui est nécessaire pour évaluer les données de mesure déterminées,
une zone de référence comprenant seulement une partie du composant et le procédé (100) étant mis en œuvre sous forme d'étalonnage en ligne, et
l'étape (106) de détermination d'au moins un paramètre géométrique comportant au moins une des sous-étapes suivantes (118, 120, 122) :
- déterminer (118) les positions des zones de référence les unes par rapport aux autres dans la représentation de composant numérique, déterminer des positions projetées des zones de référence dans au moins une partie des données de mesure déterminées et utiliser les positions des zones de référence et les positions projetées des zones de référence pour déterminer au moins un paramètre géométrique ; et/ou
- simuler (120) une mesure en transmission sur la représentation de composant numérique et/ou une segmentation basée sur la représentation de composant numérique, la simulation générant des données de mesure simulées, et utiliser une comparaison des zones de référence dans les données de mesure déterminées et les données de mesure simulées pour déterminer au moins un paramètre géométrique ; et/ou
- faire varier (122) au moins un paramètre géométrique de la représentation de composant numérique dans au moins une projection de la représentation de composant numérique, déterminer au moins un paramètre de qualité dans au moins une zone de référence d'une reconstruction du composant utilisant l'au moins une projection, répéter la variation dudit au moins un paramètre géométrique et la détermination d'au moins un paramètre de qualité dans au moins une zone de référence jusqu'à ce qu'au moins un paramètre géométrique optimisé ait un paramètre de qualité qui soit une valeur maximale de tous les paramètres de qualité déterminés, et utiliser l'au moins un paramètre géométrique optimisé comme au moins un paramètre géométrique déterminé,
**caractérisé en ce que**
à l'étape (104), il est déterminé pour chaque zone de référence quelle sous-étape (118, 120, 122) peut être utilisée pour déterminer les paramètres géométriques à l'aide de la zone de référence.

2. Procédé selon la revendication 1, **caractérisé en ce que** l'au moins un paramètre géométrique décrit une géométrie d'acquisition utilisée pour la mesure en transmission du composant.

3. Procédé selon la revendication 1 ou 2, **caractérisé en ce que** le procédé (100) comporte, entre l'étape (102) de détermination des données de mesure, et l'étape (104) d'identification de zones dans la représentation de composant numérique et/ou dans la géométrie de composant comme zones de référence, l'étape suivante :
- déterminer (108) une reconstruction préliminaire du composant comme représentation de composant numérique à partir d'au moins une partie des données de mesure déterminées au moyen d'au moins un paramètre de départ géométrique prédéfini,
à l'étape (104) d'identification de zones dans la représentation de composant numérique et/ou dans la géométrie de composant comme zones de référence, les zones étant identifiées dans la représentation de composant numérique et/ou dans une segmentation basée sur la représentation de composant numérique.

4. Procédé selon la revendication 3, **caractérisé en ce que** le procédé (100) comporte, après l'étape (106) de détermination d'au moins un paramètre géométrique, en outre les étapes suivantes :
- répéter (110) les étapes suivantes tant qu'un critère d'interruption prédéfini n'est pas satisfait :
- déterminer (112), au moyen de l'au moins un paramètre géométrique, une autre reconstruction préliminaire du composant comme représentation de composant numérique à partir d'au moins une partie des données de mesure déterminées ;
- identifier (114) des zones dans la représentation de composant numérique comme zones de référence ;
- déterminer (116), au moyen des zones de référence, au moins un autre paramètre géométrique qui remplace l'au moins un paramètre géométrique et qui est nécessaire pour évaluer les données de mesure.

5. Procédé selon l'une des revendications 1 à 4, **caractérisé en ce que**, à la sous-étape (118) de détermination des positions des zones de référence les unes par rapport aux autres dans la représentation de composant numérique, de détermination de positions projetées des zones de référence dans au moins une partie des données de mesure déterminées et d'utilisation des positions des zones de référence et des positions projetées des zones de référence pour déterminer au moins un paramètre géométrique, les géométries des zones de référence sont prises en compte lors de la détermination des positions projetées.

6. Procédé selon l'une des revendications 1 à 4, **caractérisé en ce que**, dans la sous-étape (120) de simulation d'une mesure en transmission sur la représentation de composant numérique et/ou de la segmentation basée sur la représentation de composant numérique, la simulation générant des données de mesure simulées, et d'utilisation d'une comparaison des zones de référence dans les données de mesure déterminées et les données de mesure simulées pour déterminer au moins un paramètre géométrique, les données de mesure simulées sont générées uniquement pour des zones de la représentation de composant numérique qui comportent des zones de référence.

7. Procédé selon l'une des revendications 1 à 4, **caractérisé en ce que**, dans la sous-étape (122) de variation d'au moins un paramètre géométrique de la représentation de composant numérique dans au moins une projection de la représentation de composant numérique, de détermination d'au moins un paramètre de qualité dans au moins une zone de référence d'une reconstruction du composant qui utilise l'au moins une projection, de répétition de la variation de l'au moins un paramètre géométrique et de la détermination d'au moins un paramètre de qualité dans au moins une zone de référence jusqu'à ce qu'au moins un paramètre géométrique optimisé ait un paramètre de qualité qui soit une valeur maximale de tous les paramètres de qualité déterminés, et d'utilisation de l'au moins un paramètre géométrique optimisé comme au moins un paramètre géométrique déterminé, seules les zones de la représentation de composant numérique sont à nouveau reconstruites et analysées au moyen des paramètres géométriques ayant varié dans la représentation de composant numérique, qui comportent des zones de référence.

8. Procédé selon l'une des revendications 1 à 7, **caractérisé en ce que** l'étape (104) d'identification de zones dans la représentation de composant numérique et/ou dans la géométrie de composant comme zones de référence, comporte la sous-étape suivante :
- segmenter (124) des interfaces de la représentation de composant numérique.

9. Procédé selon l'une des revendications 1 à 8, **caractérisé en ce que**, à l'étape (104) d'identification de zones dans la représentation de composant numérique et/ou dans la géométrie de composant comme zones de référence, des zones sont identifiées dans lesquelles sont disposées des inclusions de matière et/ou d'air.

10. Procédé selon l'une des revendications 1 à 9, **caractérisé en ce que**, à l'étape (104) d'identification de zones dans la représentation de composant numérique et/ou dans la géométrie de composant comme zones de référence, des zones sont identifiées qui présentent une géométrie qui est sphérique ou qui est classée comme sphérique avec prise en compte d'une zone de tolérance prédéfinie.

11. Procédé selon l'une des revendications 1 à 10, **caractérisé en ce que**, à l'étape (104) d'identification de zones dans la représentation de composant numérique et/ou dans la géométrie de composant comme zones de référence, des zones sont identifiées qui sont un objet d'un ensemble dans le composant et qui présentent un coefficient d'absorption qui est situé dans un intervalle prédéfini.

12. Procédé selon l'une des revendications 1 à 11, **caractérisé en ce que**, à l'étape (104) d'identification de zones dans la représentation de composant numérique et/ou dans la géométrie de composant comme zones de référence, des zones sont identifiées qui sont disposées dans un volume de la géométrie de composant dans lequel aucune zone de référence ou un nombre de zones de référence inférieur à une valeur limite prédéfinie n'a été identifié.

13. Procédé selon l'une des revendications 1 à 12, **caractérisé en ce que**, à l'étape (106) de détermination d'au moins un paramètre géométrique, qui est nécessaire pour évaluer les données de mesure, au moyen des zones de référence, des paramètres de sécurité des zones de référence sont pris en compte, un paramètre de sécurité indiquant le degré de sécurité d'une position de la zone de référence qui lui est associé.
